(19) Europäisches Patentamt / European Patent Office / Office européen des brevets

(11) **EP 3 154 516 B1**

(12) **EUROPEAN PATENT SPECIFICATION**

(45) Date of publication and mention
of the grant of the patent:
**04.09.2019 Bulletin 2019/36**

(21) Application number: **15734080.3**

(22) Date of filing: **15.06.2015**

(51) Int Cl.:
*A61K 38/095* (2019.01)   *A61K 9/00* (2006.01)
*A61K 47/36* (2006.01)

(86) International application number:
**PCT/EP2015/063347**

(87) International publication number:
**WO 2015/193246 (23.12.2015 Gazette 2015/51)**

(54) **STABILIZED DESMOPRESSIN**

STABILISIERTES DESMOPRESSIN

DESMOPRESSINE STABILISEE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.06.2014 KR 20140073067**

(43) Date of publication of application:
**19.04.2017 Bulletin 2017/16**

(73) Proprietor: **Ferring B.V.**
**2132 JX Hoofddorp (NL)**

(72) Inventors:
• **LEE, Bong Sang**
**Uiwang-si**
**Gyeonggi-do (KR)**
• **PARK, Su-Jun**
**Yongin-si**
**Gyeonggi-do 448-504 (KR)**
• **HAN, Jiyeong**
**Ulsan 682-800 (KR)**

• **KIL, Myeongcheol**
**Iksan-si**
**Jeollabuk-do 570-741 (KR)**
• **KIM, Min Seop**
**Seoul 130-782 (KR)**

(74) Representative: **Weidner Stern Jeschke**
**Patentanwälte Partnerschaft mbB**
**Universitätsallee 17**
**28359 Bremen (DE)**

(56) References cited:
**EP-A2- 0 252 732       WO-A2-03/094886
WO-A2-2007/083323**

• **PRAJAPATI VIPUL D ET AL: "Pharmaceutical
applications of various natural gums, mucilages
and their modified forms", CARBOHYDRATE
POLYMERS, APPLIED SCIENCE PUBLISHERS,
LTD. BARKING, GB, vol. 92, no. 2, 15 November
2012 (2012-11-15), pages 1685-1699,
XP028972872, ISSN: 0144-8617, DOI:
10.1016/J.CARBPOL.2012.11.021**

**Description**

**FIELD OF THE INVENTION**

The present invention relates to pharmaceutical compositions in the form of orally disintegrating films comprising desmopressin or a pharmaceutically acceptable salt thereof as an active ingredient, wherein the desmopressin or pharmaceutically acceptable salt thereof is stabilized in the pharmaceutical composition, to methods for stabilizing desmopressin or a pharmaceutically acceptable salt thereof in a pharmaceutical composition in the form of an orally disintegrating film, and to methods for preparing orally disintegrating films comprising desmopressin or a pharmaceutically acceptable salt thereof.

**BACKGROUND OF THE INVENTION**

Desmopressin is a synthetic analogue of the natural antidiuretic hormone vasopressin.

Unlike vasopressin, desmopressin has no vasopressor activity, but only has antidiuretic activity. The selective antidiuretic activity is due to its ability to bind to V-2 receptors only and not to V-1 receptors. V-2 receptors are G-protein coupled receptors present in the collecting ducts of the kidney and are responsible for the promotion of water reabsorption via stimulation of cyclic AMP production. Desmopressin is effective in treatment of various urinary disorders, such as, but not limited to diabetes insipidus, incontinence, enuresis and nocturia, and dysfunctions of the coagulative system. In particular, desmopressin is useful in abnormal too frequent urination, particularly nocturnal polyuria, (passing of large volumes of urine at night but normal amounts during the day) which is the main cause of primary nocturnal enuresis (involuntary passage of urine during sleep) and nocturia (the complaint that the individual has to wake at night one or more times for urination).

Most of the existing medicines used in the treatment of nocturnal enuresis and nocturnal polyuria are taken with water. Accordingly, there is a continuous need for further pharmaceutical preparations for treating urinary disorders such as nocturnal enuresis and nocturnal polyuria, which minimize the intake of water.

Preparation of medications that will avoid the intake of water such as film preparations requires methods of manufacturing using relatively high temperatures, in particular during drying of the formulation to prepare the film. This may create drawbacks for the stability of the active agent desmopressin. Desmopressin (*1-desamino-8-D-arginine vasopressin*) is a peptide containing nine amino acids. Peptides generally tend to denature, i.e. lose their native state structure when for example external stress(es) is applied, when brought into contact with a compound(s) such as a strong acid or base, a concentrated inorganic salt, or an organic solvent (e.g., alcohol or chloroform), or e.g. when exposed to radiation or heat. Therefore, desmopressin is vulnerable to instability during and/or after medicine preparation, because of its tendency to denature, in particular due to thermal denaturation.

WO 03/094886 A2 discloses stable orodispersible forms with desmopressin and fish gelatin for treatment of nocturnal enuresis. It is also mentioned that in place of fish gelatin polysaccharides, such as hydrolyzed dextran, dextrin and alginates or modified starch, may be used. It is also said that other carrier materials may be present, for example, amongst others, gums, such as tragacanth, xanthan, carrageenan, and guar.

Thus, there is a need for a desmopressin formulation that does not require water-intake and that is stable during processing, distribution, storage and preservation, and a method for the preparation thereof.

**SUMMARY OF THE INVENTION**

The subject invention provides a pharmaceutical composition in the form of an orally disintegrating film comprising an active ingredient and a stabilizing agent, wherein the active ingredient is desmopressin or a pharmaceutically acceptable salt thereof, wherein the stabilizing agent is at least one gum and wherein the weight ratio of desmopressin or a pharmaceutically acceptable salt thereof and the gum(s) ranges from 3:1 to 1:10.

The subject invention further provides for the use of one or more gums to increase the stability of a pharmaceutical composition comprising desmopressin or a pharmaceutically acceptable salt thereof as an active ingredient against denaturation, as a result of e.g. application of external stress(es) or contact with a compound(s) such as but not limited to a strong acid or base, a concentrated inorganic salt, or an organic solvent, or exposure to radiation or heat, wherein the weight ratio of desmopressin or a pharmaceutically acceptable salt thereof and the gum(s) ranges from 3:1 to 1:10.

The subject invention also provides for a method for preparing a composition according to the invention, comprising adding at least one gum to a solution comprising desmopressin or a pharmaceutically acceptable salt thereof as an active ingredient and water as the only solvent, spreading the solution onto a support and drying the spread solution to prepare an orally disintegrating film.

## DETAILED DESCRIPTION OF THE INVENTION

The subject invention provides for a pharmaceutical composition in the form of an orally disintegrating film comprising an active ingredient and a stabilizing agent, wherein the active ingredient is desmopressin or a pharmaceutically acceptable salt thereof, wherein the stabilizing agent is at least one gum and wherein the weight ratio of desmopressin or a pharmaceutically acceptable salt thereof and the gum(s) ranges from 3:1 to 1:10.

In one embodiment, the pharmaceutically acceptable salt of desmopressin is desmopressin acetate.

"Gum" as used herein should be understood to refer to hydrophilic materials that are polymers composed of heteropolysaccharides with high viscosity even at a low concentration, and are bound to water to form a viscous solution or a gel. The gum is used as a stabilizing agent for desmopressin or a pharmaceutically acceptable salt thereof. Non-limiting examples of 'gums' which can be used in the present invention are galactomannan gum (including acacia gum, locust bean gum, tara gum, and guar gum), carrageenan gum, xanthan gum, tragacanth gum, agar, quince seed gum, karaya gum, arabic gum, and gellan gum.

In a preferred embodiment, the gum is xanthan gum.

In a preferred embodiment, the composition does not substantially comprise additional stabilizing agents other than gum(s). As used herein, the term "not substantially comprise" means that the amount of additional stabilizing agents other than gum(s) is 10% (w/w) or less, 5% (w/w) or less, 4% (w/w) or less, 3% (w/w) or less, 2% (w/w) or less, 1% (w/w) or less, 0.5% (w/w) or less, and more preferably 0.1% (w/w) or less based on the total weight of all stabilizing agents used. In other words, the at least one gum constitutes at least 90%, 95%, 96%, 97%, 98%, 99%, 99.5%, or 99.9% by weight based on the total weight of all stabilizing agents used.

The pharmaceutical composition may be for use in treating or preventing various urinary disorders such as diabetes insipidus, incontinence, enuresis and nocturia, and dysfunctions of the coagulative system, in particular nocturnal enuresis or nocturnal polyuria.

It is envisaged that the pharmaceutical composition provides for increased stability of desmopressin or a pharmaceutically acceptable salt thereof against denaturation during application of external stress(es), contact with a compound(s) such as but not limited to a strong acid or base, a concentrated inorganic salt or an organic solvent, or when exposed to radiation or heat.

It is envisaged that the pharmaceutical composition will provide increased stability of desmopressin or a pharmaceutically acceptable salt thereof against thermal denaturation, in particular against thermal denaturation during drying, during distribution, during storage and/or during preservation under normal conditions, meaning, in the context of this application, room temperature (15 - 25°C) and 60% relative humidity.

The present invention further provides for the use of one or more gums to increase the stability of a pharmaceutical composition in the form of an orally disintegrating film comprising desmopressin or a pharmaceutically acceptable salt thereof as an active ingredient against e.g. denaturation by application of external stress(es), contact with a compound(s) such as, but not limited to, a strong acid or base, a concentrated inorganic salt, an organic solvent, or exposure to radiation or heat, in particular against thermal denaturation, more in particular against thermal denaturation during drying at a temperature of about 80°C for about 30 minutes or during at least 6 weeks distribution, storage and/or preservation under normal conditions, wherein the weight ratio of desmopressin or a pharmaceutically acceptable salt thereof and the gum(s) ranges from 3:1 to 1:10.

The subject invention further provides a method for preparing a composition according to the invention, comprising adding at least one gum to a solution comprising desmopressin or a pharmaceutically acceptable salt thereof as an active ingredient and water as the only solvent, spreading the solution onto a support and drying the spread solution to prepare an orally disintegrating film.

The solution used for the preparation of an orally disintegrating desmopressin film of the subject invention contains water as the sole solvent, thereby restricting the use of an organic solvent that may remain in a medicine to be administered to patients and may cause safety problems.

Generally, the use of water as the sole solvent requires drying for a long duration under high-temperature conditions, as compared with the use of an organic solvent. For peptides such as desmopressin, such long duration high temperature conditions may result in deterioration of the composition's stability due to denaturation. In the present invention, this problem has been overcome by the addition of at least one gum, thereby concomitantly also obliviating the need for the use of (toxic) organic solvents in the production process.

In the present invention, the preparation solution of an orally disintegrating film, after having being spread on a support, is preferably dried at a temperature of 100°C or less, 90°C or less, 80°C or less, preferably at about 80°C. For drying, time periods of 100 minutes or less, 50 minutes or less, 30 minutes or less, 20 minutes or less, more preferably 15 minutes or less have been proven to be appropriate so as to minimize the stability deterioration of desmopressin or a pharmaceutically acceptable salt thereof.

In other embodiments, the preparation solution of an orally disintegrating film is preferably dried at a temperature of 100 °C or less for about 30 minutes, at a temperature of 90 °C or less for about 30 minutes, at a temperature of 100 °C or less for about 15 minutes, at a temperature of 90 °C or less for about 15 minutes, at a temperature of about 80°C for about 30 minutes or at a temperature of about 80°C for about 15 minutes so as to minimize the stability deterioration of desmopressin or a pharmaceutically acceptable salt thereof.

Further, the present invention provides an orally disintegrating film prepared by the above-mentioned method. Particularly, the present invention provides an orally disintegrating film, comprising desmopressin or a pharmaceutically acceptable salt thereof as an active ingredient, in which a gum is used as a stabilizing agent for desmopressin.

In the present invention, the thickness of the orally disintegrating film may be controlled by a person having ordinary skill, but is preferably controlled to be 80 μm or less so as to minimize the drying time and obtain the physical stability of the film.

Thus, the present invention demonstrates that the use of one or more gums is specifically beneficial to increase the stability of a pharmaceutical composition comprising desmopressin or a pharmaceutically acceptable salt thereof as an active ingredient. In other words, the one or more gums are used as a stabilizing agent for stabilizing desmopressin or a pharmaceutically acceptable salt thereof against e.g. denaturation by application of external stress(es), contact with a compound(s) such as, but not limited to, a strong acid or base, a concentrated inorganic salt, or an organic solvent, or exposure to e.g. radiation or heat. In particular, the one or more gums are used as a stabilizing agent for stabilizing desmopressin or a pharmaceutically acceptable salt thereof against thermal denaturation.

"Thermal denaturation" as used herein should be understood to refer to desmopressin or a pharmaceutically acceptable salt thereof being denatured by heat during either the manufacturing process (e.g. during drying) of the pharmaceutical composition as well as under distribution, storage and preservation conditions. The pharmaceutical composition is thus stabilized against thermal denaturation e.g. during drying at a temperatures of 100°C or less, 90°C or less, 80°C or less for 100 minutes or less, 50 minutes or less, 30 minutes or less, 20 minutes or less, or 15 minutes or less. The pharmaceutical composition is also stabilized against thermal denaturation during at least 6 weeks, at least 4 weeks, at least 3 weeks or at least 2 weeks of distribution, storage and/or preservation under normal conditions.

In the present invention, the gum can effectively stabilize desmopressin or pharmaceutically acceptable salts thereof by the use of a small amount, compared to the amounts of gum(s) when used as e.g. thickening agents in pharmaceutical compositions. The weight ratio of desmopressin or a pharmaceutically acceptable salt thereof and the gum ranges from 3:1 to 1:10, most preferably from 1:1 to 1:2. When the gum is used in an amount less than the weight ratio range, desmopressin or a pharmaceutically acceptable salt thereof cannot be sufficiently stabilized. When the gum is used in an excessive amount higher than the weight ratio range, the viscosity becomes too high and it is difficult to obtain fluidity for several purposes, particularly during the manufacturing process.

In the present invention, preferably, the pharmaceutical composition comprises about 0.1 to 0.5 percent by weight of desmopressin or a pharmaceutical acceptable salt thereof, and about 0.05 to 5 percent by weight of gum(s). The term 'pharmaceutically acceptable salt' as used herein refers to any organic or inorganic addition salts which are non-toxic and have an effective function harmless to the patients, so side effects attributed to the salts do not deteriorate the beneficial efficacy of desmopressin. For example, in order to form such a salt, organic acids and inorganic acids as a free acid, or non-toxic salts may be used. Examples of the inorganic acids may include hydrochloric acid, phosphoric acid, sulfuric acid, nitric acid, and tartaric acid. Examples of the organic acids may include methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycollic acid, gluconic acid, galacturonic acid, glutamic acid, glutaric acid, glucuronic acid, aspartic acid, ascorbic acid, carbonic acid, vanillic acid, and hydroiodic acid. Among these, acetic acid is preferably used. The acid addition salts may be prepared according to any conventional method, for example, by dissolving a compound in excessive amounts of an aqueous solution of acid, and precipitating the resulting salt in a water-miscible organic solvent such as methanol, ethanol, acetone, and acetonitrile. Examples of the non-toxic salts may include sulfate, pyrosulfate, bisulfate, sulfite, bisulfite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, sebacate, fumarate, maleate, butyne-1,4-dioate, hexane-1,6-dioate, benzoate, chlorobenzoate, methyl benzoate, dinitro benzoate, hydroxybenzoate, methoxy benzoate phthalate, terephthalate, benzene sulfonate, toluene sulfonate, chlorobenzene sulfonate, xylene sulfonate, phenyl acetate, phenyl propionate, phenyl butyrate, citrate, lactate, beta-hydroxybutyrate, glycolate, malate, tartrate, methane sulfonate, propane sulfonate, naphthalene-1-sulfonate, naphthalene-2-sulfonate, and mandelate.

The pharmaceutical composition according to the present invention comprises desmopressin or a pharmaceutically acceptable salt thereof as an active ingredient, and therefore, can be used in the treatment or prevention of diseases, symptoms, and disorders that need the pharmacological effect of desmopressin or a pharmaceutically acceptable salt thereof, for example, nocturnal enuresis or nocturnal polyuria.

The term "treatment" as used herein refers to any actions that improve or favorably modify diseases, disorders and symptoms thereof by the administration of the pharmaceutical composition. Also, the term 'treatment' includes the meaning of 'prevention' broadly, so the term 'prevention' refers to any actions that inhibit diseases, disorders and symptoms thereof or suppress occurrence thereof.

The pharmaceutical composition according to the present invention may further comprise pharmaceutically acceptable carriers which are conventionally added to a pharmaceutical composition. The pharmaceutically acceptable carriers may include but are not limited to additives such as fillers, pH adjusting agents, protecting agents, wicking agents, diluents, disintegrating agents, binders, lubricants, emulsifiers, non-effervescent disintegrants, effervescent disintegrants, surfactants, anti-oxidants, wetting agents, taste-masking agents, preservatives and/or suspending agents. If necessary, sweetening agents, flavors, coloring agents and/or printing pigment colours may be further added.

When the pharmaceutical composition of the present invention is used in the treatment of urinary disorders such as nocturnal enuresis, besides desmopressin or a pharmaceutically acceptable salt thereof, other drugs may concomitantly be used unless deteriorating the object of the present invention. For example, at least one drug selected from the non-limiting examples consisting of antidiuretic hormone, tolterodine, tamsulosine, amitriphthaline, and a combination thereof may optionally be further used.

The pharmaceutical composition according to the present invention is formulated in the form of an orally dissolving film. The terms "orally dissolving film", "film", "strip" and "orally disintegrating film" are used interchangeably herein and should be understood to be administered by placing it on the tongue, under the tongue, in the oral cavity, or any other mucosal sublingual parts.

The orally disintegrating film of the present invention dissolves in less than 30 seconds i.e. fulfills the respective criteria for such type of medication both in the U.S. and Europe.

According to the present invention, the addition of gum(s) can thus effectively stabilize desmopressin or a pharmaceutically acceptable salt thereof to allow formulation in the form of a film, in particular an orally disintegrating film, thereby solving the need for water intake, and also allow for drying of the film preparation solution in which water is used as the sole solvent.

**EXAMPLES**

The invention is further described in the following examples, which are not in any way intended to limit the scope of the inventions as claimed.

Preparation Example

Preparation of a Film Formulation comprising Desmopressin as an Active Ingredient

A film formulation which has an increased stability against denaturation of desmopressin was prepared as follows:

A gum as well as further excipients (as specified in nature and amounts below in the Examples) were added to water and stirred for dissolution and dispersion, followed by homogenization using a homogenizer (Ultra Turrax T-25, IKA, 5000 rpm). Thereto, desmopressin acetate was added and dissolved, followed by homogenization again using the same homogenizer. The resulting film-preparation solution was degassed under vacuum conditions, and coated onto a polyethylene terephthalate (PET) film. The film was dried (under conditions as specified below in the Examples) to obtain a desmopressin-containing film formulation having a thickness of $80\mu$m.

Analytical Tests

The analytical tests used in the following Examples are based on <Desmopressin Acetate, USP 35> and are described in detail as follows:

Total Impurities (Examples 1, 2, 3) and Assay (Example 1)

Test solution - Examples 1, 2, 3

A film prepared according to the Preparation Example, equivalent to 1 mg of desmopressin acetate, was put in a 10 ml volume flask. It was mixed with the mobile phase as listed for the HPLC conditions hereinunder until the solution reached the marking for 10 ml. The solution was put into a centrifuge tube, and then centrifuged for 20 minutes. The solution was filtered with 0.2 $\mu$m filter (hydrophilic polytetrafluoroethylen (PTFE)). The completion of these steps resulted in the test solution (0.1 mg/ml).

Excipient solution - Examples 2, 3

The amount of each excipient (HPC, $TiO_2$, gums) in the film used for preparation of test solution was put in a 10 ml volume flask. The mobile phase as listed for the HPLC conditions hereinunder was poured into the flask until the solution reached the marking for 10 ml. The solution was put into the centrifuge tube and then centrifuged for 20 minutes. The solution was filtered with 0.2 μm filter (hydrophilic PTFE). The completion of these steps resulted in the excipient solution. This solution was used for identification of peaks originating from excipients which might show on the chromatograms. These peaks have to be distinguished from the peaks of the active ingredient and the impurities originating from denaturation thereof.

Blank - Examples 1, 2, 3

The mobile phase alone is used as a blank test solution to identify characteristics thereof to distinguish from other peaks on the chromatograms.

Standard solution - for Assay analysis - Example 1

20 mg of desmopressin acetate was taken accurately, and put in a 200 ml volume flask with mobile phase. The solution was sonicated, and then stirred for dissolution (0.1 mg/ml).

Calculation of Assay and Total Impurities

1. Assay

$$\text{Assay(\%)} = \frac{At}{As} \times \frac{Cs}{Ct} \times P$$

At : Area response of desmopressin in test sample solution
As : Area response of desmopressin in standard sample solution
Ct : Desmopressin concentration of test sample solution
Cs : Desmopressin concentration of standard sample solution
P : Purity of desmopressin acetate standard (%)

2. Total Impurities

Total Impurities = Sum of Individual Impurities

$$\text{Individual Impurity(\%)} = \frac{Ai}{At} \times 100$$

Ai : Area response of impurity in test sample solution
At : Area response of desmopressin in test sample solution

* The peaks from the blank solution and the excipient solution were excluded from the calculation of the area response of the impurity.

HPLC conditions

- Detector: UV (220 nm)

- Column : ODS (L1), 250 x 4.6mm, 5 μm

(Kromasil 100-5-C18, 250 x 4.6 mm)

- Column oven : 30°C

- Flow rate : 1.0 ml/min.

- Injection volume : 100 $\mu$l

- Mobile phase
  Buffer* : Acetonitrile (78 : 22)

- Run time : 50 min.

* Buffer solution : 3.4 g of monobasic potassium phosphate and 2.0 g of sodium 1-heptanesulfonic acid was dissolved in 1000 ml of water. The pH was adjusted to 4.50±0.05 with phosphoric acid or sodium hydroxide, as needed and passed through a filter having a porosity of 0.45 $\mu$m.

LOD (Examples 1,2)

0.5 g of the film prepared as described above in the Preparation Example was tested as follows:

- A glass bottle was dried in 105°C chamber for 1 hour and then cooled down for 30 minutes in the desiccator (room temperature).

- The cooled glass bottle from the desiccator was weighed. The film sample was then rolled or folded and then, without undue delay, put in a glass bottle in standing position. The glass bottle with the sample was weighed accurately.

- The glass bottle with the film sample was incubated in the 105°C chamber for 4 hours.

- After 4 hours, the glass bottle was cooled down for 30 minutes in the desiccator (room temperature).

- The cooled glass bottle was then weighed without undue delay.

- To calculate the LOD value, the reduced weight of the film sample was divided by the weight of the first film sample.

Example 1

Determination of the Conditions for the Film Drying

The film-preparation solutions were prepared by the same method as described in the Preparation Example, with the components and amounts as given in Table 1. The resulting film-preparation solutions were degassed under vacuum conditions, and coated on a PET film. The films were dried under different drying conditions (Temperature, Moving speed, Air flow rate) (See Table 2).

Table 1

| Composition | Amount (%) |
|---|---|
| Desmopressin acetate | 0.25 |
| Xanthan gum | 0.25 |
| Titanium dioxide | 10.00 |
| Hydroxypropyl cellulose (HPC) | 89.50 |
| Water | Q.S. |

Table 2

| Test No. | Drying condition | | Retention time in Dryer (min.) | Zone within drying chamber | | | |
|---|---|---|---|---|---|---|---|
| | | | | 1 | 2 | 3 | 4 |
| 1 | Speed(m/min) | Temperature(°C) | 10.0 | 80 | 80 | 85 | 90 |
| | 0.8 | Air flow rate(RPM) | | 1000 | 1200 | 1400 | 1700 |
| 2 | Speed(m/min) | Temperature(°C) | 10.0 | 80 | 80 | 85 | 100 |
| | 0.8 | Air flow rate(RPM) | | 1000 | 1200 | 1400 | 1700 |
| 3 | Speed(m/min) | Temperature(°C) | 10.0 | 80 | 80 | 100 | 100 |
| | 0.8 | Air flow rate(RPM) | | 1000 | 1200 | 1400 | 1700 |
| 4 | Speed(m/min) | Temperature(°C) | 13.2 | 80 | 80 | 100 | 100 |
| | 0.6 | Air flow rate(RPM) | | 1000 | 1200 | 1400 | 1700 |
| 5 | Speed(m/min) | Temperature(°C) | 10.0 | 80 | 80 | 100 | 110 |
| | 0.8 | Air flow rate(RPM) | | 1000 | 1200 | 1200 | 1700 |
| 6 | Speed(m/min) | Temperature(°C) | 13.2 | 80 | 80 | 110 | 110 |
| | 0.6 | Air flow rate(RPM) | | 1000 | 1200 | 1200 | 1700 |
| 7 | Speed(m/min) | Temperature(°C) | 13.2 | 80 | 80 | 100 | 100 |
| | 0.6 | Air flow rate(RPM) | | 1200 | 1400 | 1600 | 1700 |
| RPM : revolutions per minute | | | | | | | |

The sampled film samples (300mm X 300mm) at each set of drying conditions (Table 2) were packed in multi-layer aluminium foil, and sealed. After 6 hours, the tests namely Assay, Loss on Drying (LOD) and Total Impurities, for each sample were carried out. Assay (%) determines the amount of desmopressin maintained after film drying. Total impurities (%) determines the amount of impurities from desmopressin measured after film drying. Loss on Drying (%) is the value to measure the amount of volatile matters (in particular, water) in a film after the film is dried. For example, the LOD of 8.5 (%) of Test sample no. 1 in Table 3 indicates that the loss in weight is 8.5% of the film. The results of the tests are shown in Table 3.

Table 3

| Test No. | Retention time in a Dryer (min.) | Min./Max. temperature (°C) | Assay(%) | LOD(%) | Total Impurities (%) | Result |
|---|---|---|---|---|---|---|
| **1** | **10.0** | **80/90** | **99.5** | 8.5 | 0.61 | **Good** |
| **2** | **10.0** | **80/100** | **97.8** | 7.9 | 0.73 | **Good** |
| **3** | **10.0** | **80/100** | **100.0** | 8.0 | 0.77 | **Good** |
| **4** | **13.2** | **80/100** | **102.4** | 8.1 | 0.74 | **Good** |
| 5 | 10.0 | 80/110 | 95.6 | 8.2 | 0.75 | Poor |
| 6 | 13.2 | 80/110 | 86.0 | 6.4 | 1.04 | Poor |
| **7** | **13.2** | **80/100** | **99.2** | 6.9 | 0.67 | **Good** |

The overall result was deemed to be "Good" when the results of all three tests met the following criteria: Assay: 97.0 - 103.0%; LOD not more than (NMT) 10%; Total Impurities: NMT 1.0%. When at least one of the test results did not meet the respective criterion, the overall result was deemed to be "Poor".
Test Nos. 1 to 4 and 7 satisfied all of three conditions.
In Test Nos. 5 and 6, the LOD value was lower than 10.0%. In addition, Total Impurities (%) was also not lower than 1.0% in Test No. 6 but lower than 1.0% in Test No. 5,. However, in Test Nos. 5 and 6, the Assay value was out of the range of 97.0% to 103.0% so that the overall result for both Test No. 5 and 6 was "Poor".

From the above results, it was concluded that desmopressin acetate films cannot maintain stability if being dried at the highest temperature of 110°C for 10 minutes or more.

Example 2

The Stabilizing Effect of Gum(s)

The film was prepared according to the method as described in the Preparation Example with the components and amounts as given in Table 4. The film was dried at 80°C for 30 minutes.

Table 4

| Composition (%) | | control | Example | | | | | | | |
|---|---|---|---|---|---|---|---|---|---|---|
| | | | 1 | 2 | 3 | 4 | 5 | 6 | 7 | 8 |
| Desmopressin acetate | | 0.25 | | | | | | | | |
| Xanthan gum | | - | 0.025 | 0.050 | 0.083 | 0.250 | 0.500 | 2.500 | 7.500 | 12.500 |
| Titanium dioxide | | 10.0 | | | | | | | | |
| Hydroxypropyl cellulose (HPC) | | Q.S. | | | | | | | | |
| Water | | Q.S. | | | | | | | | |
| Total (as solid) | | to 100.0 % | | | | | | | | |
| Viscosity of solution | | 3,000 - 4,000 cp | 3,000 - 4,000 cp | 3,000 - 4,000 cp | 3,000 - 4,000 cp | 3,000 - 4,000 cp | 3,000 - 4,000 cp | 7,000 - 8,000 cp | 8,000 - 9,000 cp | > 10,000 cp |
| Loss on drying (%) | | 4.9 | 4.2 | 5.0 | 4.3 | 5.2 | 4.7 | 4.5 | 5.1 | 5.3 |
| Total impurities (%) | Before drying | ≤ 0.3 | | | | | | | | |
| | Initial (after drying) | ≤ 0.7 | ≤ 0.7 | ≤ 0.7 | ≤ 0.6 | ≤ 0.4 | ≤ 0.3 | ≤ 0.3 | ≤ 0.3 | ≤ 0.3 |
| | 2 weeks after drying (Accelerated conditions) | ≤ 1.5 | ≤ 1.0 | ≤ 0.8 | ≤ 0.8 | ≤ 0.5 | ≤ 0.5 | ≤ 0.3 | ≤ 0.3 | ≤ 0.3 |
| | 4 weeks after drying (Accelerated conditions) | ≥ 3.0 | ≤ 1.4 | ≤ 1.0 | ≤ 0.9 | ≤ 0.6 | ≤ 0.6 | ≤ 0.4 | ≤ 0.3 | ≤ 0.3 |
| 1 cp = 1 mPa.s | | | | | | | | | | |

Viscosity of solution was measured using a Brookfield viscometer. The results of the stability measurements are shown as Total Impurities (%). Total Impurities (%) determines the total amount of impurities of desmopressin measured after 2 - 4 weeks under accelerated conditions (40±2°C, Relative Humidity 75±5%).

The standard deviation of the LOD values was ±0.5% and there was no significant difference between the test groups.

As a result, as can be seen in Table 4, the gum, in this example xanthan gum, as a stabilizer, resulted in a significant reduction of the Total Impurities under accelerated storage conditions as well as immediately after film drying, compared to an identical composition without any gum (control), over the whole weight ratio range of desmopressin acetate to gum of 10:1 to 1:50. The strongest improvement in stability for very small amounts of gum added (columns 1 to 4) was seen for a weight ratio of 1:1, whereas the improvement in stability for further addition of gum (columns 5 to 8) was also existent.

Example 3

The Stabilizing Effect of Different Kinds of Gum(s)

The film was prepared according to the method as described in the Preparation Example, with the components and amounts as given in Table 5. The film was dried at 80°C for 30 minutes.

Table 5

| Ingredients | | (%) | Ingredients | | (%) |
|---|---|---|---|---|---|
| Desmopressin Acetate | | 0.25 | Desmopressin Acetate | | 0.25 |
| TiO$_2$ | | 10 | TiO$_2$ | | 10 |
| HPC | | 89.5 | HPC | | 77.26 |
| gums | Xanthan gum | 0.25 | gums | Xanthan gum | 12.49 |
| | Arabic gum | | | Arabic gum | |
| | Agar | | | Agar | |
| | Carrageenan | | | Carrageenan | |
| Water | | To 100 | Water | | To 100 |
| Total dried weight | | 100 | Total dried weight | | 100 |

The results of the stability measurements are shown as Total Impurities (%) (see Table 6). Total Impurities (%) determines the total amount of impurities of desmopressin measured after film drying ('Initial' in Table 6) and after 2 - 4 weeks under accelerated conditions (40±2°C°C, Relative Humidity 75±5%).

From the test results in Table 6, it can be seen that any kind of gum added showed a significant stabilizing effect compared to a composition without any gum added (column Control). In case that a high concentration of gum was used (weight ratio of desmopressin acetate (API) to gum of 1:50), an excellent stabilizing effect was shown, regardless of the kind of gum. However, in case that a low concentration of gum was used (weight ratio of 1:1), xanthan gum showed the most prominent stabilizing effect.

Table 6

| API: Stabilizer | Control | 1:1 | | | | 1:50 | | | |
|---|---|---|---|---|---|---|---|---|---|
| Stabilizer | Not added | Xanthan gum | Arabic gum | Agar | Carrageenan | Xanthan gum | Arabic gum | Agar | Carrageenan |
| Before drying | ≤0.3 | ≤0.3 | ≤0.3 | ≤0.3 | ≤0.3 | ≤0.3 | ≤0.3 | ≤0.3 | ≤0.3 |
| Initial (After drying) | ≤0.7 | ≤0.3 | ≤0.4 | ≤0.4 | ≤0.4 | ≤0.3 | ≤0.3 | ≤0.3 | ≤0.3 |
| 2 weeks after drying (Accelerated conditions) | ≤2.0 | ≤0.5 | ≤0.7 | ≤1.0 | ≤0.8 | ≤0.3 | ≤0.3 | ≤0.4 | ≤0.4 |
| 4 weeks after drying (Accelerated conditions) | ≥3.0 | ≤0.6 | ≤2.0 | ≤2.0 | ≤2.0 | ≤0.3 | ≤0.5 | ≤0.6 | ≤0.5 |

**Claims**

1. A pharmaceutical composition in the form of an orally disintegrating film comprising an active ingredient and a stabilizing agent wherein the active ingredient is desmopressin or a pharmaceutically acceptable salt thereof, wherein the stabilizing agent is at least one gum and wherein the weight ratio of desmopressin or a pharmaceutically acceptable salt thereof and the gum(s) ranges from 3:1 to 1:10.

2. The pharmaceutical composition according to claim 1 for use in treating or preventing nocturnal enuresis or nocturnal polyuria.

3. The pharmaceutical composition according to claim 1 or the composition for use according to claim 2, which is stabilized against denaturation.

4. The pharmaceutical composition according to claim 3, which is stabilized against thermal denaturation.

5. The pharmaceutical composition according to claim 4, which is stabilized against thermal denaturation during drying.

6. The pharmaceutical composition according to claim 4, which is stabilized against thermal denaturation during distribution, storage and/or preservation under normal conditions.

7. The pharmaceutical composition according to any of the preceding claims, wherein the pharmaceutically acceptable salt is desmopressin acetate.

8. The pharmaceutical composition according to any of the preceding claims, wherein the gum is xanthan gum.

9. The pharmaceutical composition according to any of the preceding claims, wherein the weight ratio of desmopressin or a pharmaceutically acceptable salt thereof and the gum(s) ranges from 1:1 to 1:2.

10. The pharmaceutical composition according to any of the preceding claims, wherein the composition comprises about 0.1 to 0.5 percent by weight of desmopressin or a pharmaceutical acceptable salt thereof, and about 0.05 to 5 percent by weight of gum(s).

11. The pharmaceutical composition according to any of the preceding claims, wherein the orally disintegrating film has a thickness of 80$\mu$m or less.

12. Use of one or more gums to increase the stability of a pharmaceutical composition in the form of an orally disintegrating film comprising desmopressin or a pharmaceutically acceptable salt thereof as an active ingredient against denaturation, wherein the weight ratio of desmopressin or a pharmaceutically acceptable salt thereof and the gum(s) ranges from 3:1 to 1:10.

13. Use according to claim 12, wherein the pharmaceutical composition is stabilized against thermal denaturation.

14. Use according to claim 13, wherein the pharmaceutical composition is stabilized against thermal denaturation during drying at a temperature of about 80°C for about 30 minutes or during at least 6 weeks distribution, storage and/or preservation under normal conditions.

15. A method for preparing a pharmaceutical composition of any of claims 1 to 11, comprising adding at least one gum to a solution comprising desmopressin or a pharmaceutically acceptable salt thereof as an active ingredient and water as the only solvent, spreading the solution onto a support and drying the spread solution to prepare an orally disintegrating film.

16. The method according to claim 15, wherein the drying is carried out at a temperature of 100°C or less.

17. The method according to claim 16, wherein the drying is carried out at a temperature of about 80°C.

**Patentansprüche**

1. Pharmazeutische Zusammensetzung in der Form eines sich oral auflösenden Films, die einen aktiven Inhaltsstoff und ein Stabilisierungsmittel umfasst, wobei der aktive Inhaltsstoff Desmopressin oder ein pharmazeutisch annehmbares Salz davon ist, wobei das Stabilisierungsmittel wenigstens ein Gummi ist und wobei das Gewichtsverhältnis von Desmopressin oder einem pharmazeutisch annehmbaren Salz davon und dem(den) Gummi(s) im Bereich von 3:1 bis 1:10 liegt.

2. Pharmazeutische Zusammensetzung nach Anspruch 1 zur Verwendung bei der Behandlung oder Verhinderung von nächtlicher Enurese oder nächtlicher Polyurie.

3. Pharmazeutische Zusammensetzung nach Anspruch 1 oder Zusammensetzung zur Verwendung nach Anspruch 2, die gegen Denaturierung stabilisiert ist.

4. Pharmazeutische Zusammensetzung nach Anspruch 3, die gegen thermische Denaturierung stabilisiert ist.

5. Pharmazeutische Zusammensetzung nach Anspruch 4, die gegen thermische Denaturierung während der Trocknung stabilisiert ist.

6. Pharmazeutische Zusammensetzung nach Anspruch 4, die gegen thermische Denaturierung während der Verteilung, Lagerung und/oder Konservierung unter normalen Bedingungen stabilisiert ist.

7. Pharmazeutische Zusammensetzung nach einem der vorangehenden Ansprüche, wobei das pharmazeutisch annehmbare Salz Desmopressinacetat ist.

8. Pharmazeutische Zusammensetzung nach einem der vorangehenden Ansprüche, wobei der Gummi Xanthangummi ist.

9. Pharmazeutische Zusammensetzung nach einem der vorangehenden Ansprüche, wobei das Gewichtsverhältnis von Desmopressin oder einem pharmazeutisch annehmbaren Salz davon und dem(den) Gummi(s) im Bereich von 1:1 bis 1:2 liegt.

10. Pharmazeutische Zusammensetzung nach einem der vorangehenden Ansprüche, wobei die Zusammensetzung etwa 0,1 - 0,5 Gew.-% Desmopressin oder eines pharmazeutisch annehmbaren Salzes davon und etwa 0,05 - 5 Gew.-% Gummi(s) umfasst.

11. Pharmazeutische Zusammensetzung nach einem der vorangehenden Ansprüche, wobei der sich oral auflösende Film eine Dicke von 80 $\mu$m oder weniger besitzt.

12. Verwendung eines oder mehrerer Gummis, um die Stabilität einer pharmazeutischen Zusammensetzung in der Form eines sich oral auflösenden Films gegen Denaturierung zu erhöhen, die Desmopressin oder ein pharmazeutisch annehmbares Salz davon als einen aktiven Inhaltsstoff umfasst, wobei das Gewichtsverhältnis von Desmopressin oder einem pharmazeutisch annehmbaren Salz davon und dem(den) Gummi(s) im Bereich von 3:1 bis 1:10 liegt.

13. Verwendung nach Anspruch 12, wobei die pharmazeutische Zusammensetzung gegen thermische Denaturierung stabilisiert ist.

14. Verwendung nach Anspruch 13, wobei die pharmazeutische Zusammensetzung gegen thermische Denaturierung während Trocknung bei einer Temperatur von etwa 80°C für etwa 30 Minuten oder während wenigstens 6 Wochen Verteilung, Lagerung und/oder Konservierung unter normalen Bedingungen stabilisiert ist.

15. Verfahren zur Herstellung einer pharmazeutischen Zusammensetzung nach einem der Ansprüche 1 bis 11, das das Zugeben von mindestens einem Gummi zu einer Lösung, die Desmopressin oder ein pharmazeutisch annehmbares Salz davon als einen aktiven Inhaltsstoff und Wasser als das einzige Lösemittel umfasst, Verteilen der Lösung auf einem Träger und Trocknen der verteilten Lösung umfasst, um einen sich oral auflösenden Film herzustellen.

16. Verfahren nach Anspruch 15, wobei die Trocknung bei einer Temperatur von 100°C oder weniger durchgeführt wird.

**EP 3 154 516 B1**

**17.** Verfahren nach Anspruch 16, wobei die Trocknung bei einer Temperatur von etwa 80°C durchgeführt wird.

**Revendications**

**1.** Composition pharmaceutique sous forme d'un film à dissolution orale comportant un une substance active et un agent stabilisant, où la substance active est de la desmopressine ou l'un de ses sels pharmaceutiquement acceptables, où l'agent stabilisant est au moins une gomme et où le rapport pondéral entre la desmopressine ou l'un de ses sels pharmaceutiquement acceptables et la ou les gomme(s) est compris entre 3:1 et 1:10.

**2.** Composition pharmaceutique selon la revendication 1 destinée à être utilisée pour le traitement ou la prévention de l'énurésie nocturne ou la polyurie nocturne.

**3.** Composition pharmaceutique selon la revendication 1 ou composition pour l'utilisation selon la revendication 2 stabilisée contre la dénaturation.

**4.** Composition pharmaceutique selon la revendication 3 stabilisée contre la dénaturation thermique.

**5.** Composition pharmaceutique selon la revendication 4 stabilisée contre la dénaturation thermique pendant le séchage.

**6.** Composition pharmaceutique selon la revendication 4 stabilisée contre la dénaturation thermique pendant la distribution, le stockage et/ou la conservation dans des conditions normales.

**7.** Composition pharmaceutique selon l'une quelconque des revendications précédentes, où le sel pharmaceutiquement acceptable est l'acétate de desmopressine.

**8.** Composition pharmaceutique selon l'une quelconque des revendications précédentes, où la gomme est une gomme xanthane.

**9.** Composition pharmaceutique selon l'une quelconque des revendications précédentes, où le rapport pondéral entre la desmopressine ou l'un de ses sels pharmaceutiquement acceptables et la ou les gomme(s) est compris entre 1:1 et 1:2.

**10.** Composition pharmaceutique selon l'une quelconque des revendications précédentes, où la composition comporte environ 0,1 à 0,5 pourcent en poids de desmopressine ou de l'un de ses sels pharmaceutiquement acceptables, et environ 0,05 à 5% en poids de gomme(s).

**11.** Composition pharmaceutique selon l'une quelconque des revendications précédentes, où le film à dissolution orale présente une épaisseur de 80 $\mu$m ou moins.

**12.** Utilisation d'une ou plusieurs gommes pour augmenter la stabilité contre la dénaturation d'une composition pharmaceutique sous forme de film à dissolution orale comportant de la desmopressine ou l'un de ses sels pharmaceutiquement acceptables en tant que substance active, où le rapport pondéral entre la desmopressine ou l'un de ses sels pharmaceutiquement acceptables et la ou les gomme(s) est compris entre 3:1 et 1:10.

**13.** Utilisation selon la revendication 12, où la composition pharmaceutique est stabilisée contre la dénaturation thermique.

**14.** Utilisation selon la revendication 13, où la composition pharmaceutique est stabilisée contre la dénaturation thermique pendant le séchage à une température d'environ 80°C pendant environ 30 minutes, ou pendant au moins 6 semaines de distribution, stockage et/ou conservation dans des conditions normales.

**15.** Procédé de préparation d'une composition pharmaceutique selon l'une quelconque des revendications 1 à 11, comportant les étapes consistant à ajouter au moins une gomme à une solution comportant de la desmopressine ou l'un de ses sels pharmaceutiquement acceptables en tant que substance active et de l'eau comme unique solvant, étaler la solution sur un support et sécher la solution étalée afin de préparer un film à dissolution orale.

13

**16.** Procédé selon la revendication 15, où le séchage est réalisé à une température de 100°C ou moins.

**17.** Procédé selon la revendication 16, où le séchage est réalisé à une température d'environ 80°C.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03094886 A2